# EUROPÄISCHE PATENTANMELDUNG

(11) **EP 1 184 011 A2**
(43) Veröffentlichungstag der Anmeldung: **06.03.2002**
(21) Anmeldenummer: 01120976.4
(22) Anmeldetag: 31.08.2001
(51) Int. Cl.: A61F 9/02

(54) **Sportschutzbrille**

(30) Priorität: 02.09.2000 DE 20015200 U
(71) Anmelder: Schmidt-Betschel, Wolfram, 34537 Bad Wildungen (DE)
(72) Erfinder: Schmidt-Betschel, Wolfram, 34537 Bad Wildungen (DE)
(74) Vertreter: Freiherr von Schorlemer, Reinfried, Dipl.-Phys.

(57) **Zusammenfassung**

Es wird eine Sportschutzbrille mit einem ein Schutzglas (5) tragenden Schutzglashalter (1) beschrieben, der mit einer zu seiner Positionierung bestimmten Nasenstütze (8, 9, 10), zwei an seitlichen Verbindungsstellen (3) angebrachten Seitenbügeln (4) und einem oberen Quersteg (2) versehen ist. Erfindungsgemäß ist der Quersteg (2) zur Anlage an einer oberhalb der Augenbrauen (11) liegenden Stelle der Stirn ausgebildet und eingerichtet (Fig. 2).

## Beschreibung

Die Erfindung betrifft eine Sportschutzbrille mit einem ein Schutzglas tragenden Schutzglashalter, der mit einer zu seiner Positionierung bestimmten Nasenstütze, zwei an seitlichen Verbindungsstellen angebrachten Seitenbügeln und einem oberen Quersteg versehen ist.

Das Tragen von Sportschutzbrillen dieser Art ist bei vielen verschiedenen Sportarten erwünscht, insbesondere um Augenverletzungen, Beschädigungen von unter der Schutzbrille zu tragenden Korrekturgläsern oder eine Reizung der Augen durch äußere Einflüsse wie Zug oder Körperschweiß zu vermeiden. Das gilt unabhängig davon, ob es sich um Hallensportarten wie z. B. Hand- oder Basketball oder um auch oder nur im Freien ausgeübte Sportarten wie z. B. Rad- oder Skifahren handelt.

Ein Problem beim Tragen von Sportschutzbrillen besteht darin, daß sie häufig zu einer nicht unbeachtlichen Einschränkung des Sichtfeldes führen. Das gilt insbesondere dort, wo die Sportler aufgrund einer vorgegebenen Körperhaltung dazu gezwungen sind, nicht durch zentrale Bereiche, sondern durch Randbereiche des Schutzglases zu blicken. Ein Beispiel hierfür ist der Radrennsport, bei dem der Sportler mit weit nach vorn gebeugtem Oberkörper auf dem Rennrad sitzt und trotz des gesenkten Kopfes eine freie Geradeaus-Sicht benötigt. Wird diese durch den Schutzglashalter behindert und ist das Sehvermögen auch zu den Seiten hin eingeschränkt, werden z. B. Kurven mit verschlechterter Leistung durchfahren oder Entfernungen und Abstände falsch eingeschätzt, wodurch eine erhöhte Unfallgefahr entsteht. Ähnliche Probleme ergeben sich bei der Ausübung anderer Sportarten, bei denen ein möglichst großer Rundumblick gewünscht wird. Hinzu kommt, daß die meisten Sportschutzbrillen keinen ausreichenden Schutz gegen Zug oder das Eindringen von Körperschweiß in die Augen bieten.

Zur Verbesserung des Sichtfeldes sind Sportbrillen der eingangs bezeichneten Gattung bekannt (DE 195 30 337 A1), die eingebaute Spiegelsysteme aufweisen. Bei solchen Brillen ist das Sichtfeld allerdings von der Einstellung des Spiegelsystems abhängig und daher nicht für jede Körper- bzw. Kopfhaltung gleich gut. Außerdem ist die Herstellung derartiger Brillen vergleichsweise aufwendig.

Daneben ist es bekannt, herkömmliche Brillen mit einem Schwitzschutz zu versehen (DE 295 19 043 U1), bei dem es sich im wesentlichen um einen gegebenenfalls mit Kühlungsbohrungen versehenen Schwitzschutzsteg handelt, der durch einen Schnapp-, Steck- oder Schraubenmechanismus mit dem Brillengestell verbunden oder mit diesem in einem Teil hergestellt werden kann. In der Praxis haben sich derartige Hilfsmittel bisher jedoch nicht durchgesetzt.

Der Erfindung liegt daher das technische Problem zugrunde, die Sportschutzbrille der eingangs bezeichneten Gattung mit einfachen Mitteln so zu gestalten, daß sie einen guten Rundumblick bietet und das Anbringen eines wirksamen Schwitzschutzes ermöglicht.

Zur Lösung dieser Aufgabe ist die erfindungsgemäße Sportschutzbrille dadurch gekennzeichnet, daß der Quersteg zur Anlage an einer oberhalb der Augenbrauen liegenden Stelle der Stirn ausgebildet und eingerichtet ist.

Der obere Quersteg des Schutzglashalters der erfindungsgemäßen Sportschutzbrille liegt zweckmäßig an einer oberhalb der Augenbrauen liegenden Stelle so an der Stirn des Trägers an, daß er nahezu vollständig außerhalb des üblichen Sichtfeldes liegt und daher bei keiner Kopf- oder Körperhaltung das Sichtfeld einschränkt. Aus diesem Grund ist es auch ohne Beeinträchtigung des Sichtfeldes möglich, an der Rückseite des Querstegs einen zwischen diesem und der Stirn zu liegen kommenden, wirksamen Schwitzschutz in Form einer saugfähigen Auflage od. dgl. vorzusehen. Schließlich ermöglicht der hochgezogene Quersteg das Anbringen der Seitenbügel der Brille an außerhalb des Sichtfeldes liegenden Stellen, wodurch der gewünschte Rundumblick weiter verbessert wird.

Weitere vorteilhafte Merkmale der Erfindung ergeben sich aus den Unteransprüchen.

Die Erfindung wird nachfolgend in Verbindung mit der beiliegenden Zeichnung an einem Ausführungsbeispiel näher erläutert. Es zeigen:
Fig. 1 eine erfindungsgemäße Sportschutzbrille von innen;
Fig. 2 die Sportschutzbrille nach Fig. 1 von vom und im angelegten Zustand;
Fig. 3 eine schematische, seitlich-perspektivische Ansicht der Sportschutzbrille nach Fig. 1 und 2 in einem verkleinerten Maßstab; und
Fig. 4 eine schematische Darstellung des mit der Sportschutzbrille nach Fig. 1 bis 3 erreichbaren Sichtfeldes.

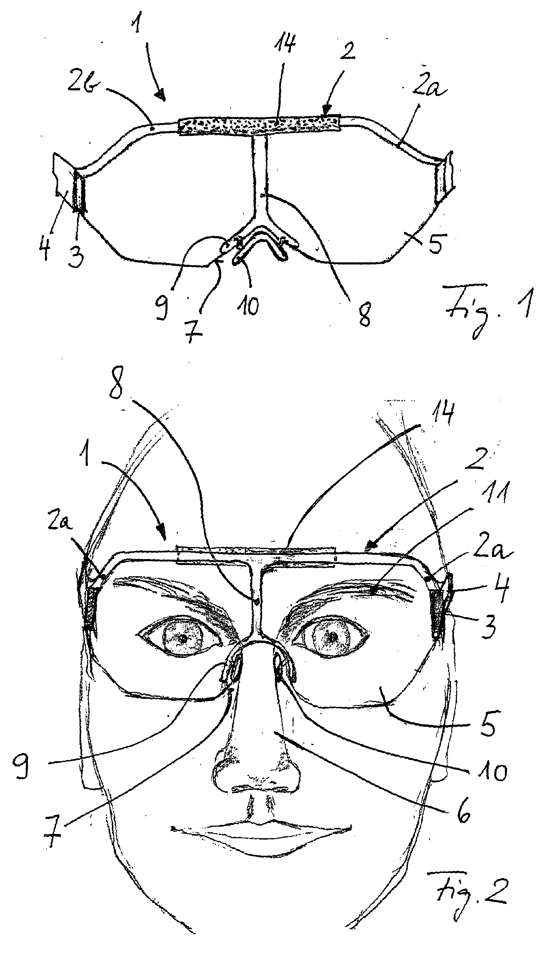

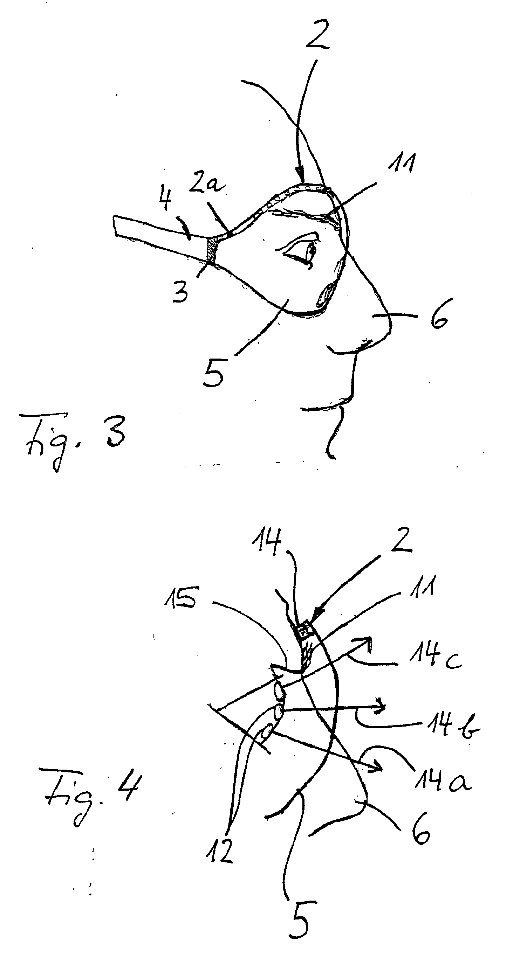

Nach Fig. 1 und 2 enthält eine erfindungsgemäße Sportschutzbrille einen Schutzglashalter 1 mit zwei seitlich angeordneten, durch einen Quersteg 2 fest miteinander verbundenen Verbindungsstellen 3, an denen wie bei üblichen Brillen zwei seitlich angeordnete Seitenbügel 4 befestigt sind. Die Verbindungsstellen 3 sind vorzugsweise als übliche Anlenkstellen bzw. Scharnierteile ausgebildet, an denen die Seitenbügel 4 schwenkbar befestigt sind. Die Seitenbügel 4 können, wie nicht näher dargestellt ist, in der Länge verstellbar und als Normal- oder Sportbügel ausgebildet sein.

Am Quersteg 2 und gegebenenfalls auch an den Verbindungsstellen 3 und weiteren, hier weniger bedeutsamen Teilen des Schutzglashalters 1 ist ein transparentes, bei Bedarf getöntes Schutzglas 5 vorzugsweise auswechselbar befestigt, das z. B. zwischen den Verbindungsstellen 3 durchgehend ausgebildet ist und in einem unteren, mittleren Bereich eine zur Aufnahme einer z. B. in Fig. 2 angedeuteten Nase 6 des Trägers bestimmte, V-förmige Ausnehmung 7 aufweist. An der Rückseite des Schutzglases 5 kann der Schutzglashalter 1 einen mittleren, vorzugsweise senkrecht zum Quersteg 2 verlaufenden und fest mit diesem verbundenen Mittelsteg 8 aufweisen, an dem zwei an den unteren Rand der Ausnehmung 7 grenzende, auf der Rückseite des Schutzglases 5 befindliche, nach Art eines umgekehrten V angeordnete Haltestege 9 angebracht sein können. An einander zugewandten Innenflächen dieser Haltestege 9 sind zweckmäßig sogenannte Pads 10 (Nasenauflagen) befestigt, die aus einem weichen, flexiblen Material bestehen und dem Zweck dienen, die Schutzbrille weich auf der Nase 6 des Trägers abzustützen. Die Haltestege 9, der Mittelsteg 8 und die Pads 10 bilden gemeinsam eine Nasenstütze der Schutzbrille, wobei diese Nasenstütze aber auch in jeder anderen zweckmäßigen Weise ausgebildet sein kann.

Erfindungsgemäß ist der Quersteg 2 so ausgebildet und angeordnet, daß der beim bestimmungsgemäßen Tragen der Schutzbrille am Kopf des Trägers an einer oberhalb der Augenbrauen befindlichen Stelle der Stirn zu liegen kommt. Dies macht insbesondere Fig. 2 deutlich, wo die Augenbrauen mit dem Bezugszeichen 11 angedeutet sind, und wird z. B. dadurch erreicht, daß der Quersteg 2 an seinen Enden über nach unten gebogene Abschnitte 2a (Fig. 1 und 2) mit den Verbindungsstellen 3 für die Seitenbügel 4 verbunden ist. Dadurch ist der Quersteg 2 in einem mittleren und insbesondere oberhalb der Augen des Trägers angeordneten Abschnitt 2b gegenüber den Seitenbügeln 4 erhöht angeordnet. Anders als bei Anwendung herkömmlicher Brillen liegt daher auch der obere Rand des Schutzglases 5 deutlich oberhalb der Augenbrauen 11, so daß der Träger der Schutzbrille in jeder Kopf- bzw. Augenstellung nur durch das Schutzglas 5 blickt und der Quersteg 2 selbst unsichtbar bleibt. Dies ist vor allem aus Fig. 4 ersichtlich, in der mit dem Bezugszeichen 12 schematisch die Iris eines Auges und mit Pfeilen 14a, 14b und 14c mögliche Blickrichtungen angedeutet sind. Dabei stellen die Pfeile 14a und 14c die durch - Bewegungen des Auges erreichbaren, oberen und unteren Grenzstellungen der Iris 12 dar, woraus erkennbar wird, daß insbesondere auch in der oberen Extremstellung (Pfeil 14c) keine Beeinträchtigung des freien Blicks durch den Quersteg 2 erfolgt. Mit dem Bezugszeichen 15 ist in Fig. 5 außerdem der obere Rand der Augenhöhle angedeutet.

Nach den Seiten hin und nach unten kann das Schutzglas 5 um die Schläfen herum bzw. bis unter die Augen des Brillenträgers herab soweit erstreckt sein, wie es zur Ermöglichung einer ungestörten Rundumsicht zweckmäßig ist. Dies ergibt sich vor allem aus Fig. 3. Danach ist der Schutzglashalter 1 so geformt bzw. gebogen, daß der Quersteg 2 und die Verbindungsstellen 3 für die Seitenbügel 4 bei bestimmungsgemäß angelegter Schutzbrille außerhalb des Sehfeldes der Augen angeordnet sind. Erreicht wird dies erfindungsgemäß insbesondere dadurch, daß der Schutzglashalter 1 bzw. der Quersteg 2 so gebogen und das Schutzglas 5 derart konvex gewölbt ist (vgl. auch Fig. 4), daß die Verbindungsstellen 3 seitlich an den Schläfen und hinter der Augenebene zu liegen kommen und daher für die Augen unsichtbar sind. Auch zu den Seiten hin ergibt sich daher ein optimales Sichtfeld.

Auf der Rückseite bzw. Innenseite des Querstegs 2 und insbesondere in einem mittleren Teil 2b davon ist vorzugsweise eine Auflage 14 aus einem gut saugfähigen Material wie z. B. Schaum- oder Textilstoff angeordnet. Die Auflage 14 ist bei angelegter Schutzbrille zwischen der Stirn des Brillenträgers und dem Quersteg 2 angeordnet (Fig. 4). Die Länge der Auflage 14 kann, symmetrisch zu beiden Seiten des Mittelstegs 8, z. B. 75 % der Länge des Querstegs 2 betragen. Dadurch wird eine verbesserte und direkte Schweißabsaugung für den auf der Stirn entstehenden Schweiß erhalten. Ist die Länge der Auflage 14 kleiner als die des Querstegs 2, entstehen beidseitig von ihr zusätzlich Lüftungsschlitze. Infolge dieser Konstruktion sammelt sich im Bereich der Augenbrauen 11 weniger Schweiß an, der sonst auf das Schutzglas tropfen und dadurch die Sicht erheblich behindern könnte. Durch Wahl der parallel zum Mittelsteg 8 gemessenen Breite und der senkrecht dazu gemessenen Höhe der Auflage 14 sowie die Lage des Querstegs 2 oberhalb der Augenbrauen 11 kann eine Optimierung der Schweißabsaugung ohne Beeinträchtigung des Sichtfeldes erzielt werden.

Ein wesentlicher Vorteil der Erfindung besteht somit darin, daß der nach oben verlegte Quersteg 2 des Schutzhalters 1 keine Beschränkung des Sichtfeldes bewirkt. Außerdem ermöglicht der Quersteg 2 einen wirksamen Schweißschutz, wobei die erhöhte Lage des Querstegs 2 sicherstellt, daß auch die Auflage 14 keine Beeinträchtigung des Sichtfeldes mit sich bringt (Fig. 4).

Bei einem praktischen Ausführungsbeispiel der Erfindung sollte der Quersteg 2 bzw. die an seiner Rückseite angebrachte Auflage 14 an einer etwa 10 mm bis 20 mm oberhalb der Augenbrauen liegenden Stelle des Trägers der Sportschutzbrille anliegen. Dies wird z. B. dadurch erreicht, daß bei einer Sportschutzbrille für eine mittlere Kopfgröße der Mittelsteg 8 eine Länge von ca. 20 mm bis 30 mm, vorzugsweise etwa 25 mm erhält bzw. der mittlere Abstand der Haltestege 9 vom Quersteg 2 auf ca. 30 mm bis 40 mm, vorzugsweise auf etwa 35 mm festgelegt wird. Je nach individueller Kopfgröße können aber auch davon abweichende Maße gewählt werden, wobei es natürlich auch zweckmäßig sein kann, unterschiedliche Brillengrößen bereit zu stellen, um dadurch den unterschiedlichen vorkommenden Kopfgrößen Rechnung zu tragen. Abgesehen davon sollte stets sichergestellt sein, daß die im Einzelfall vorgesehenen Maße die aus Fig. 4 ersichtliche Funktion beim Tragen der Sportschutzbrille ermöglichen.

Als Materialien für die beschriebenen Teile können alle diese Zwecke üblichen Materialien, insbesondere Kunststoff für den Schutzglashalter 1, den Quersteg 2 und den Mittelsteg 8 sowie Kunststoff oder Glas für das Schutzglas 5 verwendet werden, wobei das Schutzglas 5 auch beliebig getönt sein kann.

Die Erfindung ist nicht auf das beschriebene Ausführungsbeispiel beschränkt, das auf vielfache Weise abgewandelt werden kann. Dies gilt insbesondere für die Form und/oder die spezielle Konstruktion des Schutzglashalters 1, der auch anders als dargestellt ausgebildet sein könnte. Unter der Bezeichnung "Halter" sollen dabei alle Träger für Schutzgläser 5 unabhängig davon verstanden werden, ob sie als umlaufende Einfassungen bzw. Rahmen oder als einfache Stege, Klammern od. dgl. ausgebildet sind. Dasselbe gilt für die Seitenbügel 4, die vorzugsweise in der Länge verstellbar sind und anders ausgebildet sein können, als in der Zeichnung dargestellt ist. Außerdem könnte die erhöhte Lage des Querstegs 2 des Schutzglashalters 1 auch ganz oder teilweise durch eine entsprechende Formgebung für die Seitenbügel 4 erreicht werden. Weiter kann das Schutzglas 5 in an sich bekannter Weise aus einem glasklaren Material bestehen, gegen ein zweites getöntes Schutzglas über einen flexiblen Mechanismus der aufnehmenden Fassung austauschbar sein oder mit Druckknöpfen od. dgl. zum Aufclipsen mit einem zweiten, getönten Schutzglas kombiniert werden. Schließlich versteht sich, daß die einzelnen Merkmale der erfindungsgemäßen Sportschutzbrille auch in anderen als den dargestellten und beschriebenen Kombinationen angewendet werden können.

## Patentansprüche

1. Sportschutzbrille mit einem ein Schutzglas (5) tragenden Schutzglashalter (1), der mit einer zu seiner Positionierung bestimmten Nasenstütze (8, 9, 10), zwei an seitlichen Verbindungsstellen (3) angebrachten Seitenbügeln (4) und einem oberen Quersteg (2) versehen ist, **dadurch gekennzeichnet, daß** der Quersteg (2) zur Anlage an einer oberhalb der Augenbrauen (11) liegenden Stelle der Stirn ausgebildet und eingerichtet ist.

2. Sportschutzbrille nach Anspruch 1, **dadurch gekennzeichnet, daß** der Quersteg (2) zumindest in einem mittleren Abschnitt (2b) gegenüber den Verbindungsstellen (3) für die Seitenbügel (4) erhöht angeordnet ist.

3. Sportschutzbrille nach Anspruch 1 oder 2, **dadurch gekennzeichnet, daß** der Quersteg (2) an seiner Innenseite mit einer saugfähigen Auflage (14) versehen ist.

4. Sportschutzbrille nach Anspruch 3, **dadurch gekennzeichnet, daß** sich die Auflage (14) nur über einen mittleren Abschnitt (2b) des Querstegs (2) erstreckt.

5. Sportschutzbrille nach einem der Ansprüche 1 bis 4, **dadurch gekennzeichnet, daß** der Schutzglashalter (1) so geformt ist, daß der Quersteg (2) und die Verbindungsstellen (3) für die Seitenbügel (4) beim Tragen der Schutzbrille außerhalb des Sichtfeldes der Augen angeordnet sind.

6. Sportschutzbrille nach einem der Ansprüche 1 bis 5, **dadurch gekennzeichnet, daß** der Schutzglashalter (1) und/oder der Quersteg (2) derart gebogen und das Schutzglas (5) derart konvex gewölbt ist, daß die Verbindungsstellen (3) für die Seitenbügel (4) beim Tragen der Schutzbrille seitlich am Kopf und hinter der Augenebene angeordnet sind.
